(19) Europäisches Patentamt

European Patent Office

Office européen des brevets

(11) **EP 1 001 037 A2**

(12) **EUROPEAN PATENT APPLICATION**

(43) Date of publication:
17.05.2000 Bulletin 2000/20

(51) Int. Cl.[7]: **C12Q 1/68**

(21) Application number: **99307594.4**

(22) Date of filing: **27.09.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **28.09.1998 US 102069 P**

(71) Applicant:
**WHITEHEAD INSTITUTE FOR BIOMEDICAL RESEARCH**
**Cambridge, MA 02142 (US)**

(72) Inventor: **Lander, Eric S.**
**Cambridge, Massachusetts 02138 (US)**

(74) Representative:
**White, Martin Paul et al**
**Fry Heath & Spence,**
**The Old College,**
**53 High Street**
**Horley, Surrey RH6 7BN (GB)**

(54) **Pre-selection and isolation of single nucleotide polymorphisms**

(57)     Novel methods of reproducibly determining a limited population of polymorphisms are disclosed.

FIG. 1

**EP 1 001 037 A2**

## Description

BACKGROUND OF THE INVENTION

It is becoming clear that human susceptibility to disease and response to treatment is influenced by DNA sequence variations. Prominent examples include the role of variation in ApoE in Alzheimer's disease, CKR5 in susceptibility to infection by HIV, Factor V in risk of deep venous thrombosis, MTHFR in cardiovascular disease and neural tube defects, various cytochrome p450s in drug metabolism, and HLA in autoimmune disease.

Single nucleotide polymorphisms (SNPs) are nucleotide positions at which two alternative bases occur at appreciable frequency (>1%) in the human population, and are the most common type of human genetic variation. These polymorphisms are emerging as a critical tool for human genetics in general and pharmacogenomics in particular. There is growing recognition that large collections of mapped SNPs provide a powerful tool for human generic studies. A comprehensive collection of SNPs can be used to identify human disease susceptibility, either directly via association studies (which test for enrichment of a specific allele in susceptible individuals) or indirectly via linkage disequilibrium studies (which identify the presence of a common ancestral chromosome among susceptible individuals). Because this type of variation is at the sequence level, it also opens a window to the root causes of variation, including differences in gross morphology and biochemistry, and susceptibility to genetic diseases. SNPs can also be used to create more markers for genetic maps, or to study linkage disequilibrium or human evolution and migration.

Before SNPs can be systematically applied in such studies, however, it is necessary to create a large collection of such loci, construct maps of their genomic locations, and develop methods for large-scale genotyping. The sheer size and complexity of the genome makes isolation of SNPs cumbersome. In addition, as more polymorphisms are isolated and characterized, there exists the increasing possibility that "new" polymorphisms will be found to be identical to previously-characterized polymorphisms. Furthermore, although there is tremendous variation in the human population, the common SNPs that Likely underlie common disease constitute a finite collection of perhaps 3-6 million total variants.

A variety of approaches can be used to identify SNPs, depending on the desired locus type (*i.e.*, targeted vs. random) and allele frequency (*i.e.*, very common vs. less common). The most direct approach is the targeted resequencing of specific loci; that is, developing a PCR assay for a specific locus, reamplifying the locus from multiple samples (consisting of individuals and/or pools) and resequencing the resulting products to identify variant bases. Such resequencing can be performed, for example, by using conventional DNA sequencing. Targeted resequencing of specific loci has the advantage that it allows one to study a single locus across many chromosomes. However, targeted resequencing of specific loci has significant disadvantages. It is expensive and requires interpretation of sequence data from heterozygous samples, which is typically more problematic than that from single alleles.

Another approach is in use known sequence from a database, such as that from the Human Genome Project. Once a sequence of the human genome is known to high accuracy, SNPs can be isolated easily. One would only need to sequence a random fragment of human DNA and compare it to the corresponding human reference sequence. The map position of the fragment will be instantly known and every base that differs from the reference sequence will define a SNP. The advantage of the method is that it is technically straightforward and can be carried out at any scale. The disadvantage is that it requires the availability of a highly accurate reference sequence.

In advance of a complete human genome sequence, one can perform a whole-genome shotgun sequence of multiple individuals. If one obtains sufficient coverage, a given fragment will occur multiple times, allowing one to detect SNPs within that fragment. Weber and Myers (*Genome Res*. 7:401-409 (1997)) proposed shotgun sequencing to 10X depth from a mixture of individuals as a method to sequence the human genome and to simultaneously identify SNPs. The disadvantage of this approach is that it requires a commitment To sequence the entire genome to several-fold coverage.

Thus, it remains important to develop SNP discovery methods which sequence the same locus in multiple individuals, maximize sensitivity and specificity, and minimize labor and cost.

SUMMARY OF THE INVENTION

The present invention relaxes to a method of determining a limited population of polymorphisms in a reproducible set of nucleic acid molecules from one or more nucleic acid-containing samples. The method described herein does not require PCR and does not require *a priori* knowledge of the sequence of the nucleic acid molecule to be assessed. By limiting the number of polymorphisms under examination to a portion of the total number of polymorphisms that exist in the genome, the method overcomes many of the disadvantages inherent in identifying SNPs using whole genome sequencing approaches. Furthermore, the method allows sequence comparison of substantially the same subset of nucleic acid molecules across various nucleic acid-containing samples, because each sample will yield substantially the same limited population of nucleic acid molecule fragments if treated identically. That is, if a first and second nucleic acid-containing sample are subjected to a particular set of conditions (e.g., digestion with same same restriction endonuclease, such as *Bgl*II, subsequent size separation on an agarose gel, and selection of a particular gel band), each sample will produce substantially the same subset of nucleic acid molecules. Thus subset of nucleic acid molecules can then be assessed for the presence of polymorphisms (e.g., single nucleotide polymorphisms), with the advantage that each nucleic acid molecule is relatively small in comparison to the untreated nucleic acid molecule in the nucleic acid sample, i.e., is a portion of the original, untreated molecule.

In one embodiment, the invention relates to a method for determining a limited population of polymorphisms from nucleic acid molecules in a sample, comprising the steps of obtaining a nucleic acid-containing sample to be assessed treating nucleic acid molecules in said sample to produce nucleic acid fragments selected in a sequence-dependent manner by a method comprising fractionating said nucleic acid molecules to produce nucleic acid fragments, and selecting a subset of said nucleic acid fragments; selecting from said subset two or more nucleic acid fragments which occur at a corresponding chromosomal locus, thereby producing a pair, and identifying polymorphisms in a pair, thereby determining a limited population of polymorphisms from said nucleic acid-containing sample. In a preferred embodiment, the polymorphisms are single nucleotide polymorphisms.

In one embodiment, the nucleic acid molecule is DNA. In a preferred embodiment of the invention, each nucleic acid-containing sample is pooled from more than one individual. For example, the nucleic acid-containing sample can be pooled from individuals who share a particular trait (e.g., an undesirable trait, such as a particular disorder, or a desirable trait, such as resistance to a particular disorder).

In a preferred embodiment, the step of fractionating the nucleic acid molecules to produce nucleic acid fragments is performed by one or more restriction endonucleases (e.g., *Bgl*II, *Xho*I, *Eco*RI, *Eco*RV, *Hind*III, *Pst*I, and *Hae*III). In a preferred embodiment, the step of selecting a subset of said nucleic acid fragments is performed by separating the nucleic acid fragments on an agarose gel and selecting a particular band on the gel. Alternatively, this step can be performed using, for example, high pressure liquid chromatography (HPLC).

The invention also relates to a method for assaying a limited population of polymorphisms in a nucleic acid-containing sample from an individual, the method comprising obtaining a first nucleic acid-containing sample to be assessed; treating said nucleic acid-containing sample to produce nucleic acid fragments selected in a sequence-dependent manner by a method comprising fractionating said nucleic acid samples to produce nucleic acid fragments and selecting a subset of said nucleic acid fragments; selecting from said subset two or more nucleic acid fragments which occur at a corresponding chromosomal locus nucleic acid samples, thereby producing a pair; identifying nucleotide mismatches between corresponding nucleotides of a pair, thereby identifying polymorphisms; obtaining a second nucleic acid-containing sample from an individual to be assessed; and assaying said second mucleic acid-containing sample for said polymorphisms, thereby assaying a limited population of polymorphisms in a nucleic acid-containing sample from an individual.

In a preferred embodiment, a specific set of criteria is used to determine whether two or more nucleic acid fragments are derived from corresponding chromosomal loci (i.e., whether the fragments are a pair). For example, the criteria can comprise the steps of comparing the sequences of the two members of a proposed pair, wherein the two sequences are further analyzed if the two sequences are at least 80% identical over at least 80% of the length of the shorter of the two sequences; aligning the two sequences, wherein the two sequences are further analyzed if the the two sequences are identical over 10 or more bases within the first 50 bases and the last 50 bases of the sequences; identifying candidate single nucleotide polymorphisms, wherein the two sequences are father analyzed if the number of candidate single nucleotide polymorphisms does not exceed 1% of the total number of bases in the shorter of the two sequences, thereby producing a candidate match; repeating the described steps for all proposed pairs; and determining the number of candidate marches for the same chromosomal locus, wherein said candidate matches are accepted if said number of matches does not exceed expectations. Accepted candidate matches are considered a pair. In a preferred embodiment, expectations are determined according to binomial or Poisson distributions.

BRIEF DESCRIPTION OF THE DRAWINGS

Fig. 1 is a graph showing the proportion of SNPs identified (y-axis) as a function of the coverage (x-axis). The five curves, from bottom to top, correspond to $p$ (minor allele frequency) of 10%, 20%, 30%, 40% and 50%. The proportion of SNPs identified increases with coverage, and more common SNPs are more rapidly detected than less common ones.

Fig. 2 is a graph showing the relative efficiency (in terms of unique SNPs discovered, x-axis) of detecting a SNP having minor allele frequency $p$ as a function of the fold coverage (x-axis). The five curves, from bottom to top, correspond to $p$ of 10%, 20%, 30%, 40% and 50%.

Fig. 3 is a graph showing the expected posterior distribution of allele frequency for SNPs discovered by sampling three chromosomes. As shown by the relatively flat distribution, even tough there are more rare SNPs than commonly occurring ones, one is more likely to sample the more common SNPs than the rare ones, simply because of their higher rare of occurrence.

Fig. 4 is a graph showing the number of human restriction fragments with sizes in a 200 bp range centered am a given point, for a typical six-cutter restriction enzyme with an average fragment size of 4 kb.

Fig. 5 is a graph showing the size distribution of inserts for the *Bgl*II and the *Hind*III llibraries. Size of the inserts in bp (x-axis) is shown as a percentage of all sequence reads (y-axis). For the *Bgl*II library, the central distribution is 570 bp $\pm$ 17 bp, and 82% of the inserts fall within 2 standard deviations of the mean.

Fig. 6 is a graph showing the estimated complexity for libraries made from various fractions of a *Bgl*II digest, based on the length of the fragments examined (x-axis), and the number of sequencing reads done (y-axis).

Fig. 7 is a flow chart illustrating the steps used to process sequencing reads into pairs.

Fig. 8 is a histogram showing the Poisson-expected (black bars) and observed (white bars) percentages of the total number of reads (y-axis) that fall into groups of sizes 1 trough 10 (x-axis), for k = 1.7.

Fig. 9 is a histogram showing the expected distribution of allele frequencies based on the percentage of SNPs examined.

DETAILED DESCRIPTION OF THE INVENTION

The present invention relates to a method of determining a hinted population of polymorphisms in a reproducible set of nucleic acid molecules from one or more nucleic acid-containing samples; the method is referred to herein as "reduced representation shotgun" (RRS). By limiting the number of polymorphisms under examination to a portion of the total number of polymorphisms that exist in the genome, the method overcomes many of the disadvantages inherent in identifying SNPs using whole genome sequencing approaches. Furthermore, the method allows sequence comparison of substantially the same subset of nucleic acid molecules across various nucleic acid-containing samples, because each sample will yield substantially the same limited population of nucleic acid molecule fragments if treated identically. That is, if a first and second nucleic acid-containing sample are subjected to a particular set of conditions (e.g., digestion with the same restriction endonuclease, such as *Bgl*II. subsequent size separation on an agarose gel, and selection of a particular gel band), each sample will produce substantially the same subset of nucleic acid molecules. This subset of nucleic acid molecules can then be assessed for the presence of polymorphisms (e.g., single nucleotide polymorphisms), with the advantage that each nucleic acid molecule is relatively small in comparison to the untreated nucleic acid molecule in the nucleic acid sample, i.e., is a portion of the original, untreated molecule.

By "limited population of polymorphisms" is meant a subset of the total polymorphic loci potentially available within the nucleic acid sample. If the nucleic acid sample is total genomic DNA, for example, then a "limited population of polymorphisms" is a population of polymorphisms that represents a subset of the total number of polymorphisms present in the entire genome of the organism.

As used herein, "substantially the same" is intended to mean at least 70%, preferably 80%, more preferably 90%, and most preferably 95% (or more) identity. However, one of ordinary skill in the art will recognize that there are situations in which complete concordance between limited populations of polymorphic is not possible. For instance, when polymorphisms are isolated from the first nucleic acid fraction, and then assayed in the equivalent fraction from another individual (*i.e*., a nucleic acid fraction created by the same techniques as those used to produce the nucleic acid fraction from which the limited population of polymorphisms was first isolated), the loci found in the two fractions will differ slightly to the extent that polymorphisms exist which alter the underlying and, in general, constant property of the sample upon which the fractionation and/or separation is based, for example, the restriction fragment site or length. For instance, DNA from two individuals cut with *Eco*RI will differ if there is a nucleotide difference within an *Eco*RI site. Put another way, the very differences that are seen in RFLP studies will also be seen in practicing the present invention, if restriction enzymes are used to create the nucleic acid fractions. However, the frequency of such RFLPs is generally relatively low (estimated to be less than 1% of such fragments) and so this does not pose a significant problem; non-restriction endonuclease-based methods can be used in these instances.

Accordingly, the method of the invention comprises the steps of obtaining a nucleic acid-containing sample to be assessed; treating nucleic acid molecules in said sample to produce nucleic acid fragments selected in a sequence-dependent manner by a method comprising fractionating said nucleic acid molecules to produce nucleic acid fragments, and selecting a subset of said nucleic acid fragments; selecting from said subset nucleic acid fragments which occur at a corresponding chromosomal locus, thereby producing a pair, and identifying polymorphisms in a pair, thereby determining a limited population of polymorphisms from said nucleic acid-containing sample.

As used herein, a nucleic acid-containing sample (also referred to as nucleic acid sample or sample) is intended to include any source or sample which contains nucleic acid (e.g., which contains nucleic acid molecules). The sample can be, for example, any nucleic acid-containing biological material (including, but nor limited to, blood, saliva, hair, skin, semen, biopsy samples, and one or more cells). The sample can be obtained from any organism, including bacteria, viruses, plants, insects, reptiles and mammals (e.g., humans). The sample can contain nucleic acid from one or more individuals or organisms; that is, the sample can be from a single individual or organism or can be a pooled sample from multiple individuals or organisms.

For example, it may be desirable to pool samples from individuals or organisms who share a particular trait. The trait may be a desirable trait (e.g., an increase in a desirable attribute such as intelligence, resistance to a particular disorder or resistance to infection by a particular organism, or a decrease in an undesirable attribute such as a reduced incidence of a particular disorder), or an undesirable trait (e.g., an increase in an undesirable attribute or a decrease in a desirable attribute). Alternatively, it may be desirable to pool samples from individuals sharing a familial relationship. Nucleic acid samples can also be obtained from defunct or extinct organisms, *e.g.*, samples can be taken from pressed plants in herbarium collections, or from pelts, taxidermy displays, fossils, or other materials in museum collections. The sample can also be a sample of isolated nucleic acid molecules, e.g., isolated DNA or DNA contained in a vector. Suitable nucleic acid samples also include essentially pure nucleic acid molecules, nucleic acid molecules produced by chemical synthesis, by combinations of biological and chemical methods, and recombinantly produced nucleic acid molecules (see *e.g.*, Daugherty, B.L. *et al*. (1991) *Nucleic Acids Res*. 19(9):2471-2476; Lewis, A.P. and Crowe, J.S. (1991) *Gene* 101:297-302).

As used herein, "nucleic acid molecule" is intended to include, but is not limited to, deoxyribonucleic acid (DNA), ribonucleic acid (RNA), cDNA, nucleic acids from mammals or other animals, plants, insects, bacteria, viruses, or other organisms.

According to the method, the nucleic acid-containing sample is treated to produce nucleic acid fragments selected in a sequence-dependent manner. For example, the sample can be subjected to fractionation and selection methods which, when combined, are sequence-dependent, and produce a subset of nucleic acid molecules from the original sample. Either or both of the fractionation and selection steps can be sequence-dependent. "Sequence-dependent manner" is intended to mean that the method relies on the underlying nucleic acid sequence in accomplishing its purpose. For example, the nucleic acid sample can be fractionated (*e.g.*, in a random or sequence-dependent manner), then subjected to a selection step that is sequence-dependent (*e.g.*, based on methylation patterns), or the nucleic acid sample can be fractionated in a sequence-dependent manner (*e.g.*, with restriction endonucleases), and then a subset can be selected (*e.g.*, with agarose gels or HPLC).

As used herein, "fractionating the nucleic acid molecules" is intended to include methods which produce fragments of the nucleic acid molecules in the original sample. These fragments are generally smaller (*i.e.*, comprise fewer nucleotides) than the nucleic acid molecules in the original nucleic acid sample. This step can be performed by biochemical, mechanical or physical means. For example, suitable methods include, but are not limited to, cleavage with restriction endonucleases, shearing, exposure to ultraviolet light and exposure to radiation. Additional methods inclue, for example, techniques that target introns, exons, signal sequences, methylation, glycosylation patterns, recognition sites for DNA binding proteins, etc. For example, a nucleic acid sample can be fractionated via treatment with one or more restriction endonucleases (*e.g.*, *Bgl*II, *Xho*I, *Eco*RI, *Eco*RV, *Hind*III, *Pst*I, *Hae*III) to produce nucleic acid fragments. Preferably the selected restriction endonuclease(s) cleave the nucleic acid molecule at approximately every 2000 bases.

Examples of fractionating nucleic acid samples in a sequence-dependent manner include methods which cleave or break nucleic acid molecules in a way that is repeatable with respect to the nucleic acid sequence. Cleavage by means of one or more restriction endonucleases is a preferred example of such sequence-dependent cleavage; for example, a given restriction enzyme reliably cuts nucleic acid at a specified sequence, *e.g.*, *Eco*RI cuts at the sequence "G|AATTC". Sequence-dependent fractionation methods which do nor specifically utilize restriction endonucleases may also be useful. For example, a method that reliably cleaved nucleic acid in the vicinity of methylated regions would tend to be "sequence-dependent" because methylation patterns tend to be conserved. In addition, some proteins, such as ribozymes, can be designed to cleave nucleic acid at a desired sire. Chemicals, ultraviolet light, radiation and other methods can also be used to effect the sequence-dependent fractionation if they can be made to cleave the nucleic acid at similar chromosomal positions between different nucleic acid samples. If the fractionation step is not sequence-dependent, then the selection step should be sequence-dependent.

Suitable methods for selecting subsets of the fractionated nucleic acid molecules include, but are nor limited to, size separation such as separation on an agarose gel or via high pressure liquid chromatography (HPLC). A subset of the total fragments can then be selected by cutting out a portion of the gel and isolating the nucleic acid fragments within the cut-out portion of the gel. The selected nucleic acid fraction can be in a broad or narrow size range, *e.g.*, 10 bases to 1000 bases, or more. More preferably, the selected fraction is from about 300 base pairs to about 1000 base pairs, such as from about 380 base pairs to about 480 base pairs, from about 400 base pairs to about 500 base pairs, from about 480 base pairs to about 580 base pairs, from about 500 base pairs to about 600 base pairs, from about 540 base pairs to about 640 base pairs, from about 380 to about 640 base pairs, from about 380 to about 500 base pairs, or from about 400 to about 600 base pairs. Selection of the subset of nucleic acid fragments can also be performed in a sequence-dependent manner. For instance, mechanical shearing of nucleic acid molecules generally breaks up nucleic acid at random intervals. However, mechanical shearing, followed by selection of those fragments that contain, *e.g.*, exon-specific sequences, produces a nucleic acid fraction the composition of which is dependent on the underlying nucleic acid sequence.

This subset of nucleic acid fragments selected in a sequence-dependent manner is analyzed to produce pairs of nucleic acid fragments which occur at a corresponding chromosomal locus. That is, a fragment from a particular chromosomal locus is paired with one or more other fragments which are from the same chromosomal locus. The fragments which are paired can be two alleles from the same individual, or two or more alleles from different individuals.

In one embodiment of the invention, specific criteria are used to determine whether two or more fragments form a pair. These criteria are designed to exclude, i.e., not include as pairs, fragments which do not occur at a corresponding chromosomal locus. For example, sequences to be excluded include highly homolgous sequences, or duplicated loci (repeats), which occur at different chromosomal locations.

In one embodiment, every fragment is compared against all other fragments using analysis steps comprising: (a) comparing the sequences of the two members of a proposed pair, where the two sequences are further analyzed if the two sequences are at least 80% identical over at least 80% of the length of the shorter of the two sequences, (b) aligning the two sequences identified from (a), where the two sequences are further analyzed if the the two sequences are identical over 10 or more bases within the first 50 bases and the last 50 bases of the sequences, (c) identifying candidate single nucleotide polymorphisms in the sequences of (b), where the two sequences are further analyzed if the number of candidate polymorphisms does not exceed 1% of the total number of bases in the shorter of the two sequences, where two sequences which meet the criteria of (a) - (c) qualify as a candidate march, (d) repeating (a) - (c) for all proposed pairs, and (e) determining the number of candidate matches for a given chromosomal locus, where the candidate matches are accepted if the number of matches does not exceed expectations. In this method, the expectations can be determined, *e.g.*, according to binomial or Poisson distributions. Two fragments that meet all of the above criteria are considered a pair.

Fragments of a pair are then compared to identify polymorphisms. As used herein, a polymorphism is an allelic variation between two samples. As used herein, the term preferably refers to single nucleotide polymorphisms (SNPs), but can also include differences in proteins (*e.g.*, isozymes, blood groups, blood proteins), differences in nucleotide sequence (*e.g.*, restriction site maps), or differences in length of a stretch of nucleic acid (*e.g.*, RFLPs (restriction fragment length polymorphisms), microsatellites, STRs (short tandem repeats), SSRs (simple sequence repeats), SSLPs (simple sequence length polymorphisms), and VNTRs (variable number tandem repeats)). A polymorphism is not limited by the function or effect it may have on the organism as a whole, and can therefore include allelic differences which may also be a mutation, insertion, deletion, point mutation, or structural difference, as well as a strand break or chemical modification that results in an allelic variant. A polymorphism between two nucleic acids can occur naturally, or be caused intentionally by treatment (*e.g.*, with chemicals or enzymes), or can be caused by circumstances normally associated with damage to nucleic acids (*e.g.*, exposure to ultraviolet radiation, mutagens or carcinogens).

A "single nucleotide polymorphism," or SNP", is a difference of a single base between two homologous nucleic acids. For example, a diploid mammal having the sequence "GCTTCCG" at a particular position on one copy of chromosome 12, and the sequence "GCTACCG" at the same position on the other copy of chromosome 12, exhibits a SNP at that position, and is heterozygous for that SNP. If the individual were homozygous (*e.g.*, had two copies of the sequence "GCTTCCG"), that SNP would not be visible within a sample of that individual's DNA, but the SNP would be visible when compared to the DNA of in individual that were either hexerozygous for that SNP (*e.g.*, had the alleles "GCTTCCG" and "GCTACCG"), or were homozygous for a different allele of that SNP (*e.g.*, "GCTACCG"). The genotype of a SNP in a sample is generally accomplished by sequencing, *e.g.*, with an M13 vector.

By "determining polymorphisms" is meant that the polymorphic loci within the nucleic acid are assayed, and the differences determined between the polymorphic locus in one nucleic acid and the polymorphic locus in another nucleic acid.

For example, one way of reproducibly determining the same limited population of polymorphisms across different nucleic acid samples would be as follows: (1) nucleic acid samples from several individuals are isolated and pooled; (2) the pooled nucleic acid sample is then fractionated in a sequence-dependent manner, *e.g.*, cut with one or more restriction enzymes; (3) the fractionated nucleic acid sample is then separated by size; (4) a size fraction is selected; (5) pair of sequences from the same chromosomal locus are selected; and (6) polymorphisms are isolated from that fraction. Other nucleic acid samples that are to be tested are then treated in the same manner, and then assayed for those same polymorphisms. To identify more polymorphisms from the original sample, the process can be repeated using a different size fraction. This approach greatly reduces the possibility of re-isolation of previously-identified polymorphisms. Alternatively, instead of using a different size fraction as the source of new polymorphisms, pooled nucleic acid can be collected from individuals unrelated to the individuals previously used. Alternatively, one or more different fractionation methods may be used.

One application of the present invention comprises (i) combining total genomic DNA from multiple individuals; (ii) digesting the mixture with a restriction enzyme (*e.g.*, *Hind*III); (iii) subjecting the resulting DNA to electrophoresis on a gel; and (iv) excising a particular band which represents or includes fragments of a particular size and cloning the restriction fragments within a specific size range (*e.g.*, 500-600 bp). Such a library represents a specific subset of the genome, containing essentially the same fragments from each individual. Within this specific subset, fragments from a particular chromosomal locus are paired to facilitate comparison of nucleic acid sequences from several individuals at that locus. These pairs are then assayed for the polymorphic loci contained therein.

In the present invention, any nucleic acid-containing sample can be directly compared to any other nucleic acid sample by simply treating the second sample in the same way as the first, *e.g.*, by digesting with *Hind*III, electrophoresis on an agarose gel, and selection of the 500-600 bp fraction. The resulting nucleic acid fraction will contain substantially the same polymorphic loci as the nucleic acid fraction from the first nucleic acid sample. Nucleic acid samples from different individuals, or from different pools of individuals, if all treated similarly, will generally produce substantially similar subsets of nucleic acid fragments, and therefore similar subsets of polymorphic loci within those subsets of nucleic acid fragments.

Many uses of SNPs require: (i) the SNP's map position in the human genome, and (ii) a genotyping assay for scoring the locus in association studies. Even if the SNPs are mapped, they cannot be used without a genotyping assay. The reduced representation approach has a powerful feature that may facilitate efficient genotyping. If one wishes to genotype a new sample for 10,000 SNPs isolated from a specific size fraction (*e.g.*, *Hind*III/500-700 bp), one could restriction-digest the sample; ligate a generic linker; isolate the appropriate size fraction; and amplify by PCR using primers complementary to the generic linker. The resulting amplification products could be hybridized to an appropriate 'genotyping array'. It is known that (i) such amplicons provide a sample with significantly reduced complexity (Lisitsyn *et al*. (1993) *Science* 259:946-51) and (ii) samples with such reduced complexity can be used as efficient probes for hybridization to DNA arrays (as shown by hybridization of mRNA to expression monitoring arrays (Lockhart, D.J. *et al*. (1996) *Nature Biotech*. 14:1675-1680). This approach has the advantage that it does not require developing specific PCR assays for each of 10,000 loci.

If additional polymorphisms are required, they can be isolated from a new fraction, which is selected to differ from the previous fraction. The new fraction can differ from the previous in the technique used to fractionate the nucleic acid, the method used to select the nucleic acid fragments, or a new subset of nucleic acid fragments can be selected, *e.g.*, if the 500-600 bp *Hind*III fraction were chosen previously, then the 600-900 bp fraction can now be chosen, or a 500-600 bp *Pst*I fraction can be used. The distribution of restriction enzyme sixes is roughly uniform across the genome, with the exception of sites containing the CpG dinucleotide, and the size of restriction fragments therefore follows an exponential distribution. For a restriction enzyme with average fragment size $d$, digesting a genome of size $G$, the number of unique fragments ($D$) in the size range $[x_1,x_2]$ is estimated by:

$$D = (G/d)(e^{-x1/d} - e^{-x2/d})$$

For a typical six-cutter enzyme, the avenge fragment size ($d$) is 4 kb, and thus D [400, 600] is 33,000. This represents 16 Mb, or 0.5% of the human genome. This model presumes that all fragments in the size range are equally represented, and laboratory techniques for selecting fragments based on size may result in a skewed distribution. Further guidance for the practitioner is provided in the examples.

The invention also provides for a method for making a genotyping chip for use in assaying a limited population of polymorphisms within a sample (see, *e.g.*, U.S. Pat. Nos. 5,861,242 and 5,837,832). Once a set of polymorphisms is isolated, probes or primers for detecting those polymorphisms can be incorporated into such a chip. When it is desirable to assay an individual for the polymorphisms in the set, nucleic acid is isolated from that individual, and it can be fractionated with the same methods that were used to isolate the original set of polymorphisms. For example, if nucleic acid from 10 individuals can be pooled, cut with *Eco*RI, and the polymorphisms isolated from the 2000 bp fraction, and primers or probes for detecting those polymorphisms can be placed on a genotyping chip. The nucleic acid from an individual to be tested could also be restricted with *Eco*RI, and the 2000 bp fraction isolated, ligated to a generic primer, and amplified based upon that primer, and applied to the genotyping chip. The method of the invention therefore allows the user to concentrate study on only a limited portion of the entire spectrum of the available polymorphisms. By examining only a limited portion of the genome, this method has the added benefit of reducing cross-reactivity between unrelated genetic sites.

The methods of the present invention can be used in humans and non-humans. For example, the methods can be used to assay polymorphisms in animals for veterinary purposes. For instance, they can be used to amplify target sequences known to be associated with susceptibilities to diseases with genetic components, or to detect known generic defects in purebred animals such as dogs or horses. They can also be used to assess levels of biodiversity in populations of animals, plants, or microorganisms. The can be applied in the search for beneficial genetic components in animals and plants, both domesticated and wild, that are used for food, feed, fiber, oils, lumber, or other raw materials. They can be applied in the search for genetic components of strains of pests, parasites or disease organisms that are especially virulent to humans, plants or animals.

The methods of the invention can also be used to amplify sequences across species. For instance, chimpanzees and humans share approximately 99% sequence similarity. The methods of the invention can be used to locate those areas in which the 1% interspecific difference is located, thereby pinpointing the "evolutionary hotspots" responsible for species differentiation, and interspecific conserved regions, as well.

The methods of the invention can also be selected and used to fingerprint proprietary biological material. For example, a set of polymorphisms can be chosen corresponding to specific genotypes known to exist in a protected crop cultivar. Assays of plants can be made according to the present invention, to determine if those plants correspond to the genotype of the patented cultivar.

The invention will be further illustrated by the following non-hitting examples. The teachings of all references cited herein are incorporated herein by reference in their entirety.

EXAMPLES

Example 1: Theoretical basis of SNP sampling.

A. Identifying SNPs by Poisson sampling. If a reduced representation library from a mixture of many individuals is sequenced to k-fold coverage, the probability of identifying a SNP with minor allele frequency *p* is:

$$\sum_{i=1}^{\infty} \pi(i,k) \left[ 1 - p^i - (1-p)^i \right]$$

where $\pi(i,k)$ is the Poisson probability that the fragment containing the SNP is sampled *i* times and the bracketed term is the probability that both alleles occur in the sample.

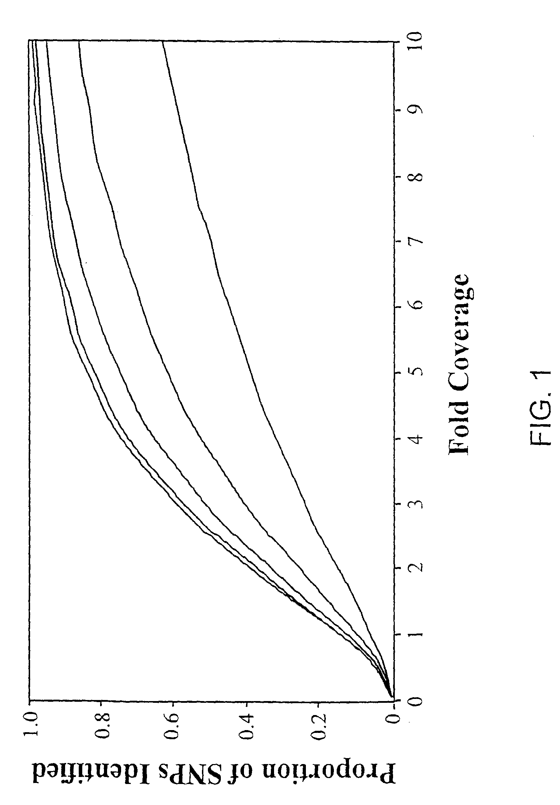

As shown in Fig. 1, the proportion of SNPs increases with coverage and more common SNPs are more rapidly detected than less common ones. Fig. 1 also shows that there are diminishing returns to deep sampling. Beyond a certain point, each additional 1x coverage yields fewer SNPs. Rather than sampling more deeply, it is more advantageous to begin sampling of a new library (*i.e.*, a new nucleic acid fraction).

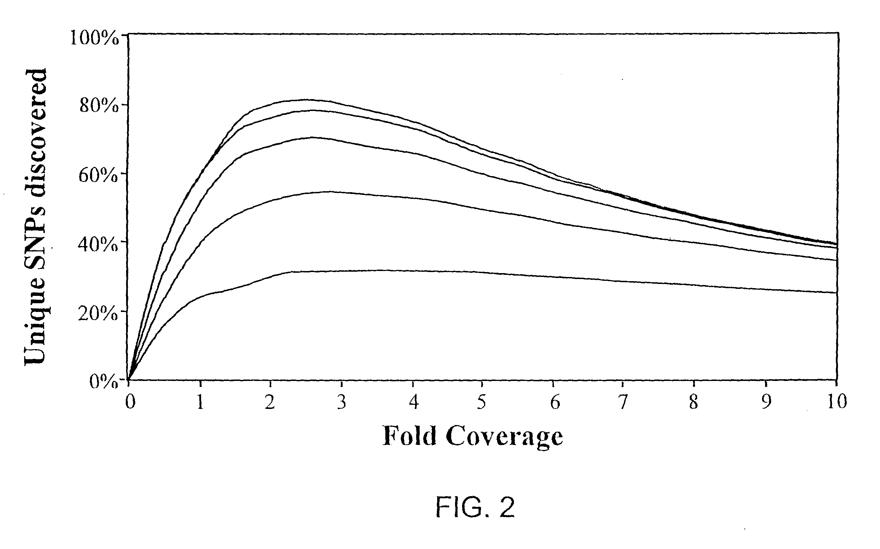

The optimal sampling depth can be determined by calculating the "efficiency", *i.e.*, the proportion of SNPs found divided by the coverage. Fig. 2 shows the relative efficiency *(i.e.*, new SNPs per read). Strikingly, the efficiency is maximized at around 2.5-fold coverage for SNPs with minor allele >20%—although the peak is relatively broad.

B. Distribution of allele frequencies. It is desirable to identity SNPs that are reasonably polymorphic in the general population, and the distribution of allele frequencies of SNPs identified in a reduced representation approach can be predicted from population genetics theory. These predictions can be compared to observed data. According to population genetics theory (Nei, M. (1987) *Molecular Evolutionary Genetics*, Columbia University Press, New York), the distribution of allele frequencies for all polymorphisms in a population follows the equation

$$F(p) = C[p(1-p)]^{\theta-1},$$

where *C* is a constant of proportionality and $\theta$ is the classical parameter $4N\mu$ (estimated by $\pi$, below). For the human population, Wang *et al*. ((1998) *Science* 280:1077-1082) have estimated $\theta$ to be approximately 0.0004.

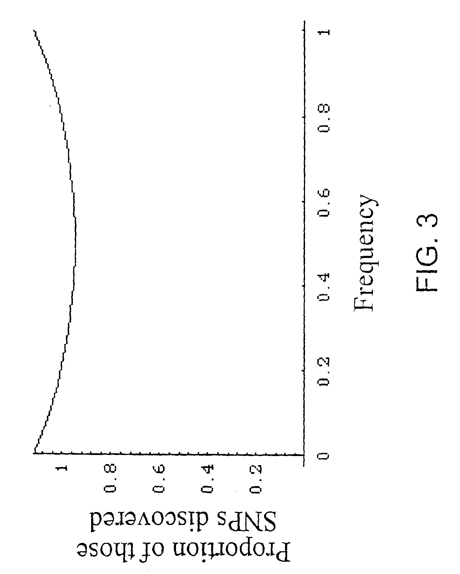

Rare alleles are less likely to be observed in a small sample. The allele frequency distribution for variants observed in a sample of i chromosomes can be determined by Bayes' theorem, using the weighting factor $[1 - p^1 - (1-p)^i]$, which reflects the chance that any given SNP will be encountered during sampling of *i* chromosomes. For SNPs found in a sample of three chromosomes, the allele frequency distribution is shown in Fig. 3, which shows that the allele frequency distribution of SNPs discovered in a small sample of chromosomes is expected to be quite flat. That is, the allele frequency of SNPs identified from a small sample is expected to be roughly uniformly distributed in the range [0,1]. The mean frequency of the minor allele is expected to be just under 25%, corresponding to heterozygosity of about 35%. These theoretical expectations agree reasonably well with the empirical finding of Wang *et al*. ((1998) *Science* 280:1077-1082). It also follows from this distribution that the maximal efficiency for identifying Common (>20%) SNPs is expected at 2-4-fold coverage. Thus, those SNPs found in a small sample are suitably biased toward having a reasonable allele frequency in the population.

C. Number of fragments in a size range. The distribution of restriction sites tends to be uniform across the human genome (with the exception of restriction sites containing the CpG dinucleotide) and thus the size of restriction fragments follows an exponential distribution. For a restriction enzyme with average fragment size $d$, the number of restriction fragments in the size range $[x_1, x_2]$ is:

$$(G/d)(e^{-x1/d} - e^{-x2/d}),$$

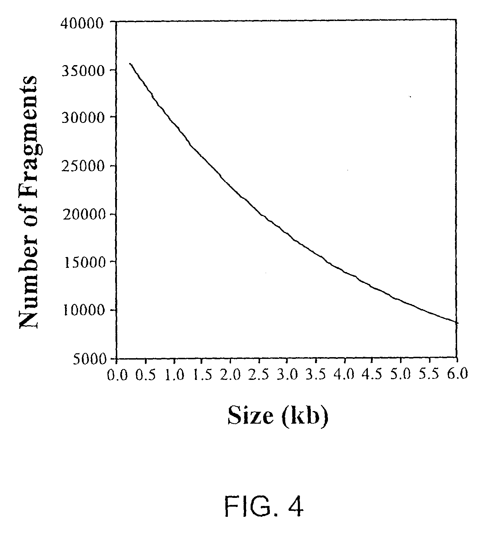

where $G$ is the genome size. For a typical six-cutter with an avenge fragment size ($d$) of about 4 kb, the number of fragments in a size window of 200 bp is shown in Fig. 4.

D. Implications. There are roughly 33,000 fragments in the range or 400 bp - 600 bp. Because such fragments could he sequenced in a single pass, it would require about 33,000k successful sequencing reads to obtain k-fold coverage. There are roughly 22,000 fragments in the range 1.9 kb - 2.1 kb. Because each fragment contains two distinct ends (of which only one is seen in a single sequencing read), there are a total of 44,000 distinct ends, and it would require about 44,000 k successful sequencing reads to obtain k-fold coverage. Reduced representation libraries are therefore of an appropriate size for discovery of SNPs. For example, obtaining 4-fold coverage would require in the range of 150,000 successful sequence reads and would survey roughly 20 Mb of genomic DNA.

E. Monitoring a library by resampling. It is not necessary to wait until 150,000 sequences have been obtained in order to test whether a reduced representation project is proceeding successfully. It is possible to monitor the success of the project by monitoring the resampling rate, *i.e.*, the frequency at which fragments are seen multiple times.

If one performs $N$ successful sequence reads from a library with $D$ distinct sequences (where $D$ is the complexity, and is either (1) the number of fragments if the fragments are small enough to be fully sequenced in a single read or (2) the number of ends if the fragments are too large to sequence in a single read), then the number of pairwise matches is $N^2/2D$. Each match will contain SNPs at a rate determined by the nucleotide diversity, $\pi$, which is defined as the per nucleotide pairwise difference between two chromosomes drawn from a population. Large-scale surveys of random DNA estimate $\pi$ at $4 \times 10^{-4}$, or 1 difference per 1200-2500 bp. Thus, in a reduced representation library containing 400-600 bp fragments, approximately 1 in 4 paired sequences should contain a SNP. It follows from the low rate of true SNPs ($5 \times 10^{-4}$) that false positives can be avoided with 95% accuracy, only if incorrect basecalls are exceedingly rare ($< 2.5 \times 10^{-5}$).

Thus, digestion of the human genome with a six-cutter restriction endonuclease, followed by size selection of 400-600 bp fragments, should result in a library containing a complexity of 30,000 - 40,000 unique genomic loci. If the library is oversampled such that individual loci are seen more than once, SNPs should be found in one out of four paired reads. If the average number of chromosomes sampled is low, the average allele frequency of the resulting variants should be biased towards highly heterozygous SNPs.

Example 2. Sample Reduced Representation Strategy

To prepare reduced representation libraries, DNA is isolated from 10-20 individuals. These ace then combined in equimolar amounts to create pooled DNA. A collection of reduced representation libraries is then prepared by digesting the DNA with a standard six-cutter enzyme (such as *Hind*III; size-fractionating it by gel electrophoresis and/or preparative HPLC; and creating a series of libraries, with each representing a distinct fraction and containing 30,000-40,000 distinct sequences.

SNPs are then identified by sequencing each library to 4.5-fold coverage. Theory suggests that the optimal depth is about 3x, although the optimum is relatively broad. Slightly deeper coverage may be appropriate to allow for imperfect fractionation. Yield should be monitored and adjusted accordingly.

A small proportion of false positives is acceptable, as these will be identified and excluded in the course of developing genotyping assays, but as the accuracy should he as high as possible, candidate SNPs should be confirmed. Past experience indicates that SNPs should be able to be identified with greater than 95% accuracy, *i.e.*, >95% of apparent SNPs will be actual SNPs. As a quality assessment measure, a subset of SNPs should be "confirmed" in order to estimate (i) accuracy and (ii) allele frequency. This can be done by testing 100 candidate SNPs by developing PCR assays; amplifying them from ten samples (*e.g.*, 7 individuals and three pools of 50 chromosomes from distinct ethnic groups), and resequencing the products to confirm the presence and frequency of the SNP.

To calculate the yield of SNPs, one can consider the following example:

| Frequency of useful SNPs found with 2-fold coverage: | 1 per 2 kb |
|---|---|
| Sequencing read length: | 500 bp |
| Sequencing pass rate: | 85% |

This implies a yield of:

$$\frac{(\text{fold coverage x frequency useful SNPs})}{(\text{sequencing read length x sequencing pass rate})}$$

or: (4.5 x 2000)/(500 x 0.85), or 1 SNP per 21.2 sequencing reads.

In general, there should be one SNP every 1000 bp, but a proportion (~1/3) will be in repetitive sequence that is suboptimal for subsequent genotyping.

Example 3. Empiric Results.

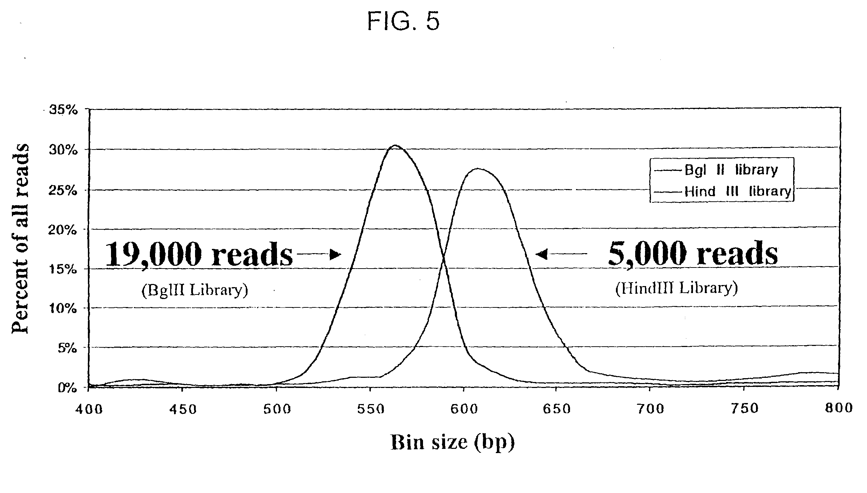

Two size-selected libraries were constructed from a diverse pool often individual humans (4 Caucasian (1 each of :Utah, French, Amish, Russian), 1 each of: Japanese, Chinese, African American, African Pygmy, Melanesian, Amerindian). The pooled DNA was digested to completion with either *Bgl*II or *Hind*III, and fragments were prepared in a narrow range around 500 bp for the *Bgl*II digestion, and around 600 bp for the *Hind*III digestion, using preparative agarose gel electrophoresis. The resulting size fractions were cloned into M13-based vectors, and individual clones were sequenced. The size distributions obtained were appropriately narrow, as is shown in Fig. 5, which is a graph showing the size distribution of inserts for the two libraries. For example, the central distribution of the *Bgl*II library had a mean insert length of 570 bp $\pm$ 17 bp. Only 84% of the sequencing reads fell within two standard deviations of the mean, as a long flat tail of contaminating sequences of various lengths was observed. This is expected, given that the sieving properties of agarose gels are known to be imperfect, with some small fragments traversing the gel more slowly than expected, and some larger fragments moving more quickly than expected.

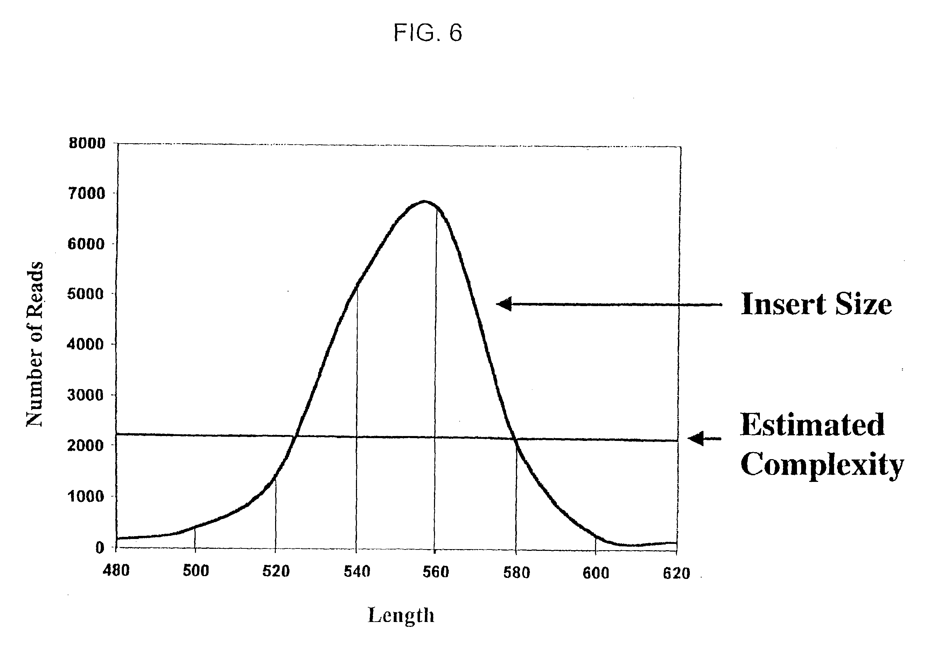

The complexity of the libraries was next determined, as the goal of reduced representation is to facilitate resampling of individual chromosomal loci. Estimated complexity for the *Bgl*II library is shown in Fig. 6, which shows the estimated complexity for libraries prepared from various size fractions (x-axis) of a *Bgl*II digest, and the number of sequencing reads done (y-axis).

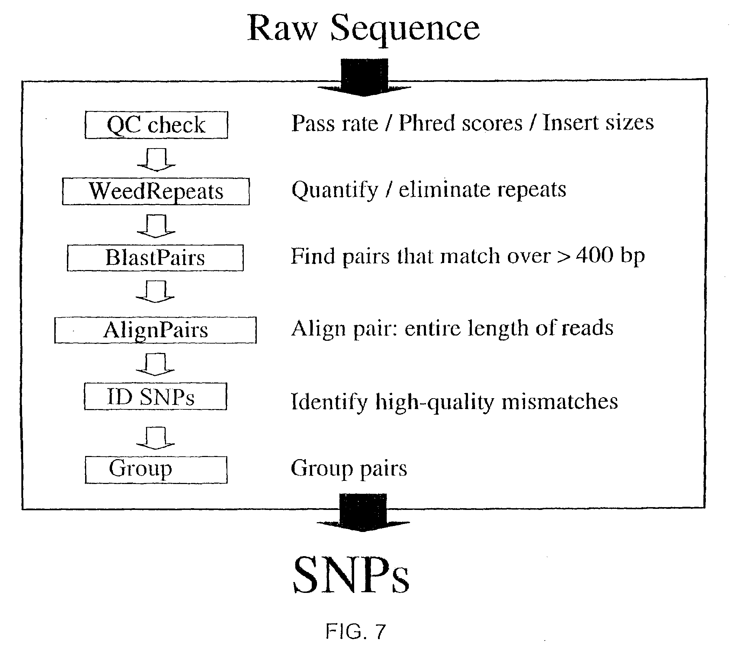

The sequencing reads were then processed as shown in Fig. 7. BLAST was first used to identify reads that were highly similar in sequence to one another, that is, the reads that had greater than 400 bp of identity, but any method of searching on the basis of similarity, and reporting on the extent of sequence similarity between pairs of reads can be used. To accurately measure the rate of resampling and find SNPs, reads must be paired only with truly orthologous sequences. The following criteria were used, after considering the expected polymorphisms between two nucleic acid fragments derived from the same locus. Once every read was compared against every other read, a pair of reads were allowed to continue through the process if, over 400 bp or more, there was 80% or more sequence identity over 80% of the length of the shorter of the two reads. Reads passing through this step were then aligned. Several criteria were applied to the aligned sequences. First, because sequence quality is often lower at the ends of reads, a 10 base pair window was examined within the first and last 50 base pairs. If the two sequences did not match perfectly within the window, the window was repeatedly shifted one base towards the middle of the alignment and the two sequences within the newly placed window were compared again. If no 10 base pair window matched within the first 50 base pairs (at either end), then the pair was not analyzed further. If there was a perfect match in a 10 base pair window within the first 50 bases of both ends, then the pair was analyzed further. This step serves to eliminate sequences with unclear sequence at either end, as well as sequences which are too short relative to each other. That is, there is no separate "trimming" step after alignment, as differences in length between two reads are viewed as a defect. The 10-base window within 50 bases of the end to work very effectively, but other sizes of windows can be used over longer distances from the ends if this is required to attain the desired sequence quality. Alternatively, this window and distance can be shortened, or this step may be eliminated altogether, if the sequence quality is deemed high enough to not require such rigorous standards.

Second, it was determined whether there were any SNPs in the pair of reads. In making this determination, quality of the sequence was also assessed. That is, differences between two reads were not assumed to be SNPs, but rather, the sequence itself was evaluated for quality, to determine if a difference was really a polymorphism, or a difference in basecalling between the two reads.

Third, since repetitive DNA was present in the libraries, it is necessary to avoid pairing sequences that originate from distinct, if homologous, genomic loci. To accomplish this, the low nucleotide diversity in the human genome ($\pi$ = 1/2000 bp) was considered, and it was concluded that any true match should have considerably less than 1% candidate SNPs. Thus, any candidate pair with > 1% high-quality discrepancies were eliminated. Specifically, the number of SNPs in an alignment were counted. If the total number of SNPs exceeded 1% of the bases, then the pair was rejected on the assumption that the two reads of the pair represented a duplicated or repetitive locus.

For example, if sequences A, B, C and D are placed in a group as possibly representing a singe locus, then each would be compared to the other. If the number of SNPs found between A and B make up less than 1% of their length, then A and B continue to be considered as being from the same locus. But if the comparison between C and D shows that SNPs make up 2.% percent of the differences between them, and either C or D, when compared to either A or B, have SNPs making up 1.2% of the differences in each comparison, then A and B are concluded to be sequences containing "true" SNPs, while C and D are considered to represent duplicated or repeated loci.

Alternatively, if one wishes to exclude all loci that are related to duplicated or repetitive loci, then the entire group of reads can be excluded.

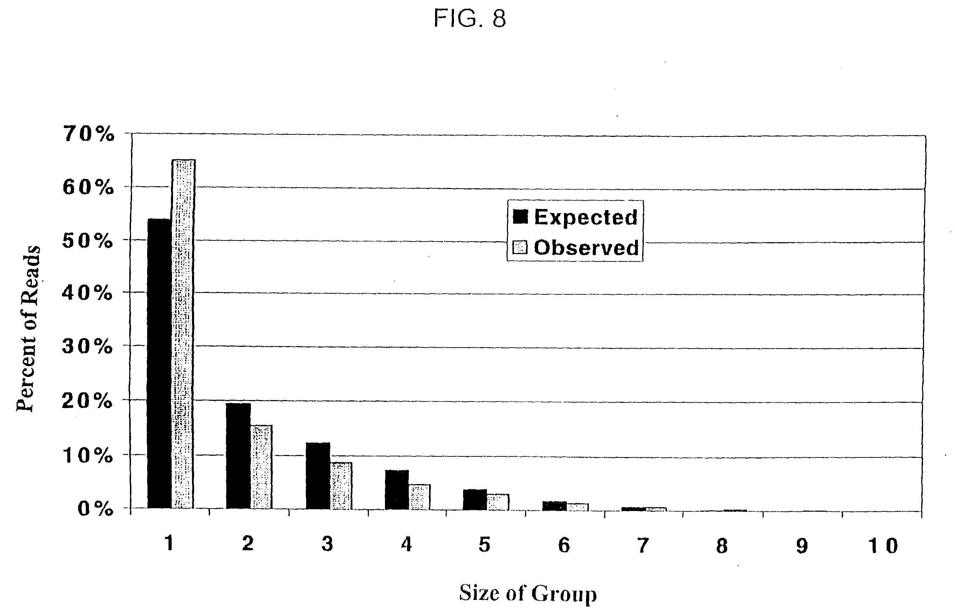

All such pairs that passed the above steps were collapsed into connected component groups, each corresponding to a putative single genomic locus. Such stringent criteria may eliminate a small number of loci that are truly highly diverse, but this was deemed to be outweighed by the concern of inappropriate pairing of non-orthologous sequences. Once paired reads were identified, the rate of matches was examined and compared to that predicted, that is, the reads were assessed for the size of their group. For a library sequenced to $k$-fold coverage, the probability that exactly $i$ orthologs of a given read are sequenced is estimated by the Poisson probability, $\pi(i,k)$. In this method, given an estimation for the number of sequences amongst the nucleic acid fragments which represent a single locus, and given a certain number of sequences examined, either the binomial or Poisson distributions can be used to determine these expectations. The Poisson distribution is shown for the *Bgl*II library in Fig. 8, which is a histogram showing the Poisson-expected (black bars) and observed (white bars) percentages of the total number of reads (y-axis) that fall into groups of sizes 1 though 10 (x-axis), for k = 1.7.

For example, groups with exactly 4 mutually matching reads (groups of exactly 4 putatively orthologous reads) are together expected to comprise about 5-10% of the total number of reads, while the reads assigned to putatively orthologous groups of size 10 involve only about 1% of all reads. Groups that are large enough that they are expected to occur less than once, based on the Poisson distribution, are discarded and non of the potential SNPs occurring between reads of these large groups are accepted.

Initial calculations modeled complexity as $D$ unique inserts, which were to be represented equally in the library. The observed size distribution was, however, skewed, as expected, due to the known imperfections of agarose gel as a sieve. That is, a band cut out of a gel in the range of 500 to 600 base pairs pairs contains fragments the sizes of which produce a bell-shaped curve, with tails extending below 500 bp and above 600 bp. The effective complexity, defined as the chance that any two reads drawn from the library would constitute a match, was then measured, and the results are show in Table 1, below.

Table 1

| Complexity of *Bgl*II and *Hind*III libraries. | | |
|---|---|---|
| Complexity = number of reads$^2$/(2 x number of pairs) , and assumes that all fragments are equally represented in the library. | | |
| Library | *Bgl*II | *Hind*III |
| Reads | 17,130 | 4,570 |
| Pairs | 14,490 | 502 |
| Complexity | 9,839 | 20,797 |
| Repeat Content | 6% | 6% |

Analysis of large numbers of clones from the *Bgl*II library revealed 14,000 paired reads, demonstrating an effective complexity of 10,000. Similarly, analysis of 23,000 clones from the *Hind*III library revealed an effective complexity of about 20,000. Furthermore, considering the skewed size distribution of reads, the rate at which reads match one another closely fits theoretical expectation, as is shown in Fig. 8, which is a histogram showing the Poisson-expected (black bars) and observed (white bars) percentages of the total number of reads (y-axis) that fall into groups of sizes 1 through 10 (x-axis) for k = 1.7.

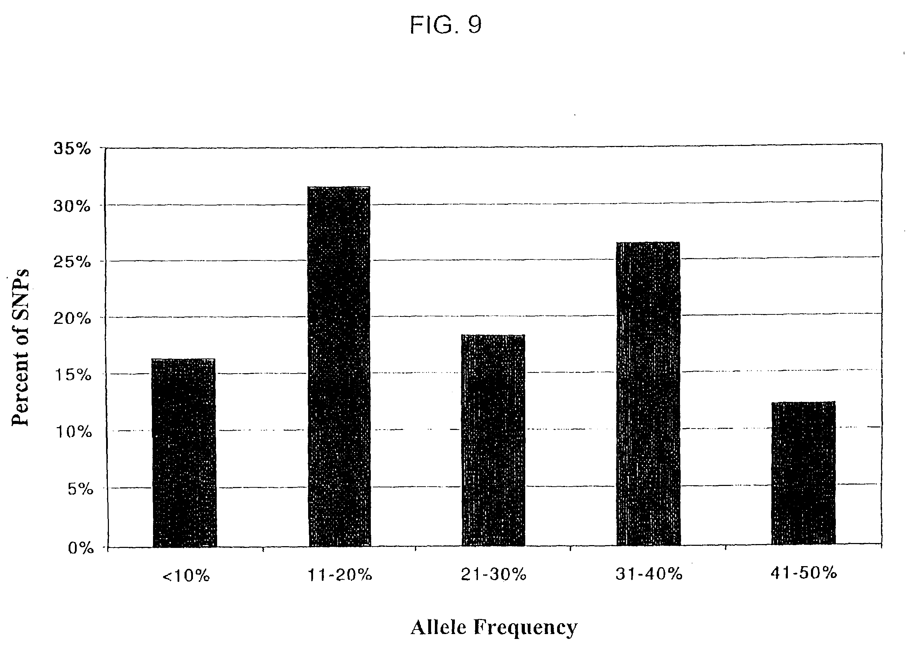

The *Bgl*II and *Hind*III libraries were shown to have the desired properties for use in the invention, producing about 1,650 SNPs from 19,000 reads, or about 1 SNP per 11 reads performed. This compares quite favorably with the results of Wang *et al*. (1998) (*Science* 280:1077-1082), in which 1 SNP was found per 12 reads for 3 DNAs screened, and 1 SNP per 48 chip hybridizations when 8 DNAS were screened. The allele frequency of these SNPs was also high, as expected from theory (Fig. 9).

All references, parents and patent applications are incorporated herein by reference in their entirety. While this invention has been particularly shown and described with references to preferred embodiments thereof, it will be understood by those skilled in the an that various changes in form and details may be made therein without departing from the scope of the invention encompassed by the appended claims.

**Claims**

1. A method for determining a limited population of polymorphisms from nucleic acid molecules in a sample, comprising the steps of:

   a. obtaining a nucleic acid-containing sample to be assessed;
   b. treating nucleic acid molecules in said sample to produce nucleic acid fragments selected in a sequence-dependent manner by a method comprising:

      i. fractionating said nucleic acid molecules to produce nucleic acid fragments; and
      ii. selecting a subset of said nucleic acid fragments;
      wherein either (i) or (ii) or both (i) and (ii) are done in a sequence-dependent manner;

   c. selecting from said subset nucleic acid fragments which occur at a corresponding chromosomal locus, thereby producing a pair, and
   d. identifying polymorphisms between fragments of a pair; thereby determining a limited population of polymorphisms from said nucleic acid-containing sample.

2. The method of Claim 1, wherein the polymorphisms are single nucleotide polymorphisms.

3. The method of Claim 1, wherein the nucleic acid-containing sample is pooled from more than one individual.

4. The method of Claim 1, wherein the nucleic acid molecules are DNA.

5. The method of Claim 3, wherein the individuals share a particular trait.

6. The method of Claim 5, where the trait is a disorder.

7. The method of Claim 1, wherein step (b)(i) is performed by one or more restriction endonucleases.

8. The method of Claim 7, wherein the one or more restriction endonucleases are selected from the group consisting of *Bgl*II, *Xho*I, *Eco*RI, *Eco*RV, *Hind*III, *Pst*I, and *Hae*III.

9. The method of Claim 1, wherein step (b)(ii) is performed using an agarose gel.

10. The method of Claim 1, wherein step (b)(ii) is performed using high pressure liquid chromatography (HPLC).

11. A method for determining a limited population of polymorphisms from nucleic acid molecules in a sample, comprising the steps of:

   a. obtaining a nucleic acid-containing sample to be assessed;

   b. treating nucleic acid molecules in said sample to produce nucleic acid fragments selected in a sequence-dependent manner by a method comprising:

      i. fractionating said nucleic acid molecules with one or more restriction endonucleases to produce nucleic acid fragments; and

ii. selecting a subset of said nucleic acid fragments using size fractionation;
wherein either (i) or (ii) or both (i) and (ii) are done in a sequence-dependent manner;

c. selecting from said subset nucleic acid fragments which occur at a corresponding chromosomal locus, thereby producing a pair, and

d. identifying polymorphisms between fragments of a pair; thereby determining a limited population of polymorphisms from said nucleic acid-containing sample.

12. The method of Claim 11, wherein the polymorphisms are single nucleotide polymorphisms.

13. The method of Claim 11, wherein the nucleic acid-containing sample is pooled from more than one individual.

14. The method of Claim 11, wherein the nucleic acid molecules are DNA.

15. The method of Claim 13, wherein the individuals share a particular trait.

16. The method of Claim 15, wherein the trait is a disorder.

17. The method of Claim 11, wherein the one or more restriction endonucleases are selected from the group consisting of *Bgl*II, *Xho*I, *Eco*RI, *Eco*RV, *Hind*III, *Pst*I, and *Hae*III.

18. The method of Claim 11, wherein step (b)(ii) is performed using an agarose gel.

19. The method of Claim 11, wherein step (b)(ii) is performed using high pressure liquid chromatography (HPLC).

20. The method of Claim 11, wherein the one or more restriction endonucleases cleave DNA on average about once every 2000 base pairs.

21. The method of Claim 11, wherein the subset of (b)(ii) is in a size range selected from the group consisting of: from about 380 base pairs to about 480 base pairs, from about 400 base pairs to about 500 base pairs, from about 480 base pairs to about 580 base pairs, from about 500 base pairs to about 600 base pairs, and from about 540 base pairs to about 640 base pain.

22. A method for assaying a limited population of polymorphisms in a nucleic acid-containing sample from an individual, the method comprising:

a. obtaining a first nucleic acid-containing sample to be assessed;
b. treating nucleic acid molecules in said sample to produce nucleic acid fragments selected in a sequence-dependent manner by a method comprising:

i. fractionating said nucleic acid molecules to produce nucleic acid fragments; and
ii. selecting a subset of said nucleic acid fragments;
wherein either (i) or (ii) or both (i) and (ii) are done in a sequence-dependent manner;

c. selecting from said subset nucleic acid fragments which occur at a corresponding chromosomal locus, thereby producing a pair;
d. identifying polymorphisms between fragments of a pair;
e. obtaining a second nucleic acid-containing sample from an individual to be assessed; and
f. assaying said second nucleic acid-containing sample for said polymorphisms,
thereby assaying a limited population of polymorphisms in a nucleic acid-containing sample from an individual.

23. The method of Claim 1, wherein step (c) is performed by the following steps:

a. comparing the sequences of the two members of a proposed pair, wherein the two sequences are further analyzed if the two sequences are at least 80% identical over at least 80% of the length of the shorter of the two sequences;

b. aligning the two sequences identified from (a), wherein the two sequences are further analyzed if the the two sequences are identical over 10 or more bases within the first 50 bases and the last 50 bases of the sequences;

c. identifying candidate single nucleotide polymorphisms in the sequences of (b), wherein the two sequences are further analyzed if the number of candidate single nucleotide polymorphisms does not exceed 1% of the total number of bases in the shorter of the two sequences, wherein two sequences which meet the criteria of (a) - (c) qualify as a candidate match;

d. repeating (a) - (c) for all proposed pairs; and

e. determining the number of candidate matches for the same chromosomal locus, wherein said candidate matches are accepted if said number of matches does not exceed expectations, wherein accepted candidate matches are considered a pair.

**24.** The method of Claim 23, wherein said expectations are determined according to binomial or Poisson distributions.

**25.** The method of Claim 22, wherein step (c) is performed by the following steps:

a. comparing the sequences of the two members of a proposed pair, wherein the two sequences are further analyzed if the two sequences are at least 80% identical over at least 80% of the length of the shorter of the two sequences;

b. aligning the two sequences identified from (a), wherein the two sequences are further analyzed if the the two sequences are identical over 10 or more bases within the first 50 bases and the last 50 bases of the sequences;

c. identifying candidate single nucleotide polymorphisms in the sequences of (b), wherein the two sequences are further analyzed if the number of candidate single nucleotide polymorphisms does not exceed 1% of the total number of bases in the shorter of the two sequences, wherein two sequences which meet the criteria of (a) - (c) qualify as a candidate match;

d. repeating (a) - (c) for all proposed pairs; and

e. determining the number of candidate matches for the same chromosomal locus, wherein said candidate matches are accepted if said number of matches does not exceed expectations, wherein accepted candidate matches are considered a pair.

**26.** The method of Claim 25, wherein said expectations are determined according to binomial or Poisson distributions.

FIG. 1

FIG. 2

EP 1 001 037 A2

FIG. 3

FIG. 4

FIG. 5

FIG. 6

Insert Size

Estimated Complexity

Number of Reads

Length

# Raw Sequence

| | |
|---|---|
| QC check | Pass rate / Phred scores / Insert sizes |
| WeedRepeats | Quantify / eliminate repeats |
| BlastPairs | Find pairs that match over > 400 bp |
| AlignPairs | Align pair: entire length of reads |
| ID SNPs | Identify high-quality mismatches |
| Group | Group pairs |

# SNPs

FIG. 7

FIG. 8

EP 1 001 037 A2

# FIG. 9

EP 1 001 037 A2